# EUROPEAN PATENT SPECIFICATION

(11) **EP 4 333 691 B1**
(45) Date of publication and mention of the grant of the patent: **08.07.2026**
(21) Application number: 22728460.1
(22) Date of filing: 09.05.2022
(51) Int. Cl.: A61B 5/00

(54) **MASSETER MUSCLE ACTIVITY MONITORING**
ÜBERWACHUNG DER AKTIVITÄT DES MUSCULUS MASSETER
SURVEILLANCE DE L'ACTIVITÉ DU MUSCLE MASSÉTER

(30) Priority: 07.05.2021 IE S20210099; 25.02.2022 IE S20220038
(43) Date of publication of application: 13.03.2024
(73) Proprietor: JAC DENTAL SOLUTIONS LIMITED, Monkstown Co. Dublin, A94 X023 (IE)
(72) Inventor: COGAN, John, Dublin, A94 X023 (IE); CURLEY, Lisa, Dublin, A94 T0V8 (IE)
(74) Representative: Purdylucey Intellectual Property
(86) International application number: PCT/EP2022/062515
(87) International publication number: WO 2022/234145

(56) References cited:
- US-A1- 2011 178 428
- US-A1- 2018 220 956
- US-A1- 2018 310 881
- US-A1- 2019 099 129
- US-A1- 2019 110 746
- US-A1- 2020 008 738

## Description

### Introduction

This invention relates to a monitoring apparatus and system for monitoring masseter muscle activity from within the buccal cavity of a wearer being a human patient or a horse, cow or other mammal.

### Background of the invention

Bruxism is a condition in which a patient clenches and grinds their teeth, being a subconscious or involuntary activity that usually occurs while the patient is asleep. Oftentimes, the patient is completely unaware that they suffer from sleep bruxism, making it difficult to diagnose and prevent. The problem of bruxism is widespread: clenching and grinding activity is excessive or problematic for 12% of the population. Yet, currently, there is no effective way of measuring the intensity, duration and times of clenching and grinding movements that characterise sleep bruxing.

Dental professionals and patients have great difficulty understanding and treating sleep bruxing as they cannot effectively measure it as it happens. A dental professional will be able to recognise the signs of bruxism by examining the resulting damage and wear to the teeth and temporal mandibular joint (TMJ) of a patient only after it has occurred.

The masseter muscle is the main muscle responsible for the articulation and movement of the mandible or lower jaw. It is known that by measuring the activity of that muscle, the movement and activity of the mandible is also measured.

It is accepted by dental professionals that electromyography (EMG) is the current gold standard for identifying bruxing events by measuring masseter muscle activity. As disclosed in WO 2018/172409 and discussed below, it is also known that masseter muscle activity can be detected by a pressure sensor, mounted on an adjacent molar within the buccal cavity, detecting the pressure exerted on the sensor as the masseter muscle expands and bears against the sensor. A third method of detecting bruxism is to use an interocclusal pressure sensor; in other words, to measure pressure exerted between opposing teeth of the upper and lower jaws. Activity of a muscle on the chin can also be detected.

Analysis of bruxism using EMG, although effective, is highly inconvenient for the patient and expensive to perform. This is because the study must be conducted during an overnight sleep in a sleep lab, supervised by a trained clinician. Also, it is known that there is a correlation between bruxism and stress experienced by a patient. Consequently, conducting bruxism analysis outside a patient's normal sleeping routine is inherently sub-optimal.

The aforementioned WO 2018/172409 discloses an oral appliance with a sensing arrangement adapted to be disposed within the buccal cavity of a patient adjacent to a muscle complex and operable to perform an action in response to movement of the muscle complex toward the sensing arrangement. Specifically, the appliance seeks to measure masseter muscle activity by reflecting a change in the girth of the masseter muscle. When the masseter muscle expands, it exerts a force on a pressure sensor of the appliance.

It is problematic to use a pressure sensor to measure masseter muscle activity. Pressure sensors require physical interaction in order to function and so depend upon contact, and upon the nature of the contact, between the muscle and the sensor. Precise positioning and orientation of a pressure sensor is therefore essential but as there are inconsistencies between patients due to physiological differences, calibration may be difficult or even impossible in some circumstances. For example, there can be variations in the direction of action of masseter muscle deformation between different patients.

Additionally, there are instances where the masseter muscle can be active but not necessarily changing significantly in girth. As pressure exerted on an adjacent pressure sensor will not change in these circumstances, the sensor will not necessarily recognise instances of muscle activity such as these.

Any protective covering or housing over the pressure sensor must be very responsive to convey the action of the masseter and therefore must be highly flexible. This makes the housing difficult to manufacture and reduces its structural integrity, including its ability to hold the sensor at a precise position and orientation.

It is also challenging to integrate pressure sensors into a nightguard for measuring interocclusal pressure between opposing teeth of the upper and lower jaws. Even if using a minimally sized pressure sensor, the patient's mandible position is likely to be altered significantly by the presence of a nightguard that is bulky enough to accommodate a sensor placed between the jaws. The resulting additional separation between the jaws is likely to influence the bruxism activity being measured and so could undermine the purpose of the measurement exercise.

US 2018/310881 and US 2019/099129 represent examples of documents that provide insight into the state of the art.

It is against this background that the present invention has been devised.

### Summary of the invention

The invention resides in a method of monitoring masseter muscle activity from a location within a patient's buccal cavity. The method comprises: placing an optical sensor into the buccal cavity, facing the masseter muscle, at a position fixed relative to at least one tooth adjacent to the masseter muscle, preferably a tooth of the upper jaw but possibly instead or additionally a tooth of the lower jaw; emitting light, such as IR light, from the optical sensor into the masseter muscle; detecting reflection of the light from the masseter muscle into the optical sensor; obtaining monitoring data from the detected light, indicative of activity of the masseter muscle; wirelessly transmitting the monitoring data to a remote receiver unit; and processing the monitoring data to filter out events involving masseter muscle activity that is irrelevant to bruxism.

The monitoring data may be processed before and/or after its transmission.

The method of the invention may further comprise placing an additional sensor onto the patient's body outside the buccal cavity or into another orifice of the patient's body, such as an ear canal. That additional sensor is one or more of an optical sensor, an accelerometer, a gyroscope, a temperature sensor, a pressure sensor, a humidity sensor and a pH sensor; and wirelessly transmitting additional data from the or each additional sensor to the remote receiver unit. The additional data can be correlated with the monitoring data against time. Conveniently, the or each additional sensor can be placed into the buccal cavity together with the optical sensor in a single unit or module.

An additional optical sensor can be placed into the buccal cavity facing another masseter muscle. Then, additional monitoring data can be obtained that is indicative of activity of that other masseter muscle. Once the additional monitoring data is transmitted wirelessly to a remote receiver unit, the monitoring data can be compared with the additional monitoring data.

Where an additional optical sensor is placed into the buccal cavity at another location or onto the patient's body outside the buccal cavity or into another orifice of the patient's body, blood pressure can be measured continuously or intermittently using data obtained from that additional optical sensor.

Bruxism is highly correlated with stress. The apparatus of the invention therefore enables better stress management by improving the ability to measure bruxing activity.

In summary, the invention exploits the hitherto undocumented insight to use an optical sensor within the mouth to measure activity of the primary muscle that controls jaw movement. EMG sensing is impractical within the mouth whereas placing an optical sensor inside the mouth to measure muscle activity brings the added advantage that the optical sensor can be fixed relative to the muscle whose activity it is measuring. This is not possible where an optical sensor is used to measure muscle activity anywhere else on the body and where, therefore, motion artefacts are a problem. Conversely, in this particular location, an optical sensor has been found to be superior to an EMG sensor and to a pressure sensor as also known in the prior art.

The optical sensor is fixed relative to the masseter muscle by being fixed relative to the upper or lower jaw by, in turn, being attached or held in fixed relation to the teeth. By thereby sensing masseter muscle activity effectively, the invention makes it possible to measure sleep stages and to categorise jaw movements. This enables tracking of digestion and mastication movements including involuntary masticating sleep bruxing movements.

Accepted wisdom in the art is that masticating movements are best measured from the masseter muscle but that sleep stages are best determined by measuring other muscles. However, in the case of measuring sleep bruxism, the inventors regard it as advantageous to determine both masticating movements and sleep stages from the masseter muscle because it is involuntary muscle movements when the user is asleep that are being measured.

The optical sensor can be totally encased or encapsulated and its function is not dependent on any physical force or contact, or on any chemical or electrical measurements. These advantages also apply to other sensors of the apparatus, such as a temperature sensor or an accelerometer, whose measurements can be used to supplement, support and corroborate measurements derived from one or more optical sensors. The advantages of the invention also apply to measurement of pH by an optical sensor.

### Brief description of the drawings

In order that the invention may be more readily understood, reference will now be made, by way of example, to the accompanying drawings in which:
Figure 1 is a schematic illustration of a masseter muscle monitoring apparatus of the invention *in situ* within a patient's buccal cavity in use, also showing a signal of masseter muscle activity produced by the sensor module;
Figure 2 is a schematic perspective view of a circuit board of the sensor module, with associated components;
Figure 3 is a graph of intensity against time during a night-time period, showing stages of sleep and the intensity, timing and duration of instances of clenching and grinding during those stages;
Figure 4 is a perspective view of an embodiment of the invention in the form of a nightguard assembly in which a sensor module is located in a housing integrated into a 3D-printed nightguard;
Figure 5 is an exploded view of the assembly shown in Figure 4;
Figures 6a to 6e are a sequence of schematic perspective views showing stages of manufacturing an apparatus of the invention by 3D printing;
Figures 7a to 7d are a sequence of schematic perspective views that show another 3D printing process for manufacturing an apparatus of the invention;
Figure 8 is a perspective view of a masseter muscle monitoring apparatus in another embodiment of the invention; and
Figure 9 is a plan view of the masseter muscle monitoring apparatus of Figure 8 *in situ* within a patient's buccal cavity in use.

### Detailed description of the preferred embodiments

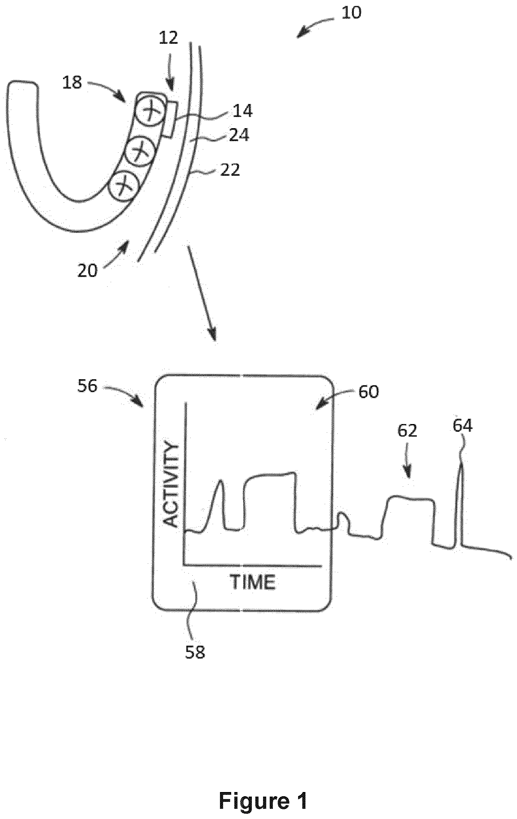

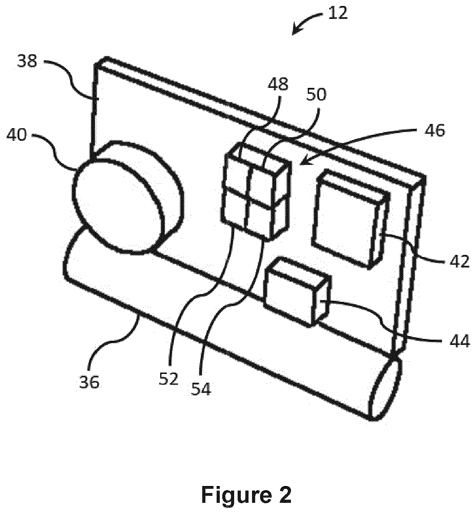

Referring initially to Figures 1 to 4 of the drawings, a masseter muscle monitoring apparatus 10 of the invention comprises a sensor unit or module 12 in which an optical sensor is connected to a transmitter, as shown in Figure 2. The sensor module 12 is mounted on, or in, a mount or housing 14 of the apparatus 10. In this example, the sensor module 12 and its close-fitting housing 14 have a shallow, approximately cuboidal profile with rounded edges that, for patient comfort, is as narrow as possible along an outward axis facing away from the teeth.

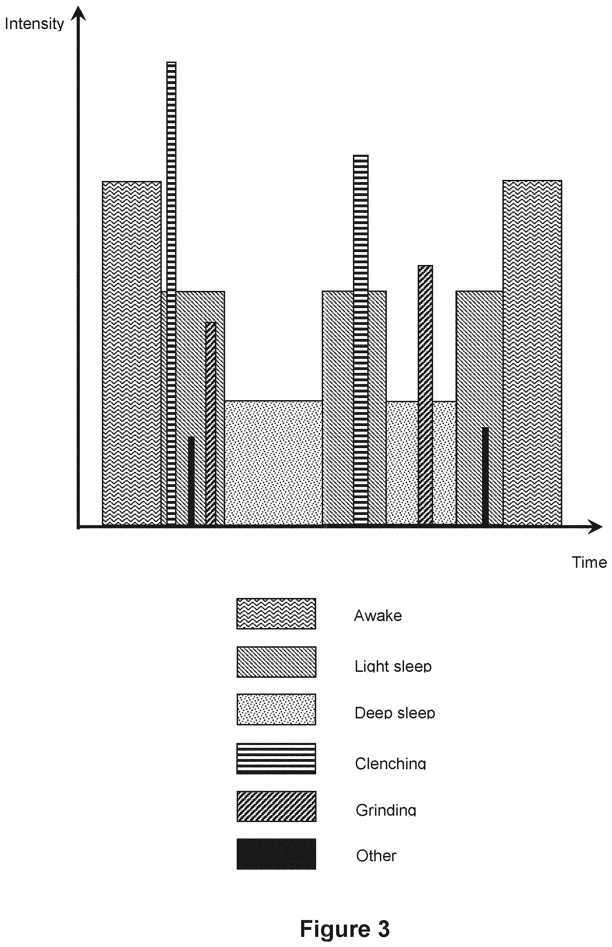

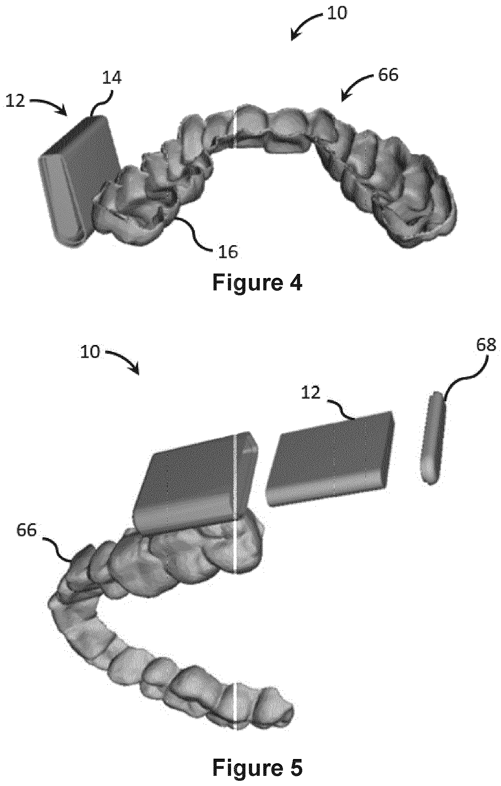

The housing 14 has a support in the form of attachment formation 16 that extends along, and is engageable with, the first and second molars 18 of the patient's upper jaw or maxilla, on either side of the mouth. As shown in the practical examples of Figures 3 and 4, the attachment formation 16 could be a mouthguard or a nightguard that engages all or most of the teeth of the patient's maxilla. For example, the attachment formation 16 could be formed with a series of complementary recesses to receive respective teeth, as shown, or could be deformable to conform to the contours of the teeth. The attachment formation 16 could instead be a smaller support such as a clip that engages fewer teeth, for example only one or two of the molars.

It will be noted from Figure 1 that the housing 14 containing the sensor module 12 is positioned within the oral vestibule 20, being the portion of the oral or buccal cavity that is situated between the patient's molars 18 and the adjacent cheek 22. Thus, the oral vestibule 20 is bounded on one side by teeth 18 and gingivae and on the other side by the patient's lips (labial vestibules) and cheeks 22 (buccal vestibules). This places the housing 14 adjacent to a masseter muscle complex 24 in the patient's cheek 22, with an optical sensor of the sensor module 12 facing the masseter muscle 24.

The sensor module 12 can be powered by a battery, by inductance, by energy from within the buccal cavity, or by any other suitable power source such as thermal energy from the patient's body. The sensor module 12 exemplified in Figure 2 comprises an onboard rechargeable battery 36. The sensor module 12 of Figure 2 further comprises a PCB 38 that supports an inductance coil 40 for contactless recharging of the battery 36. The PCB 38 also supports a processor 42, a transmitter 44 and an optical sensor 46 comprising an infra-red (IR) emitter 48 and an IR receiver 50.

The battery 36, PCB 38 and other components supported by the PCB 38 are potted or encapsulated for protection and to form the shallow cuboidal external shape of the sensor module 12 evident from Figures 1 and 4, while allowing transmission of IR light from and back to the optical sensor 46. The protective material may, for example, be polypropylene, which allows through-passage of light transmitted between the optical sensor 46 and the masseter muscle 24. The protective material and/or the material of the housing 14, which may be a similar material, seals the electronics of the sensor module 12 from the hostile environment within the buccal cavity.

In use, the IR emitter 48 illuminates the masseter muscle 24 with IR light, which penetrates effectively into the interior of the masseter muscle 24. The IR receiver 50 then receives IR light reflected from the masseter muscle 24 and produces a signal in accordance with any activity of the masseter muscle 24 that may influence the reflected IR light. The signal produced by the IR receiver is converted to digital form for transmission, processing, interpretation and display.

More specifically, the optical sensor 46 in the sensor module 12 may be a photoplethysmogram (PPG) type. PPG sensors are effective at measuring changes in blood volume in a muscle, which has a direct correlation with muscle activity. It is understood that PPG and EMG technologies produce muscle activity measurements that are of similarly high quality, but PPG sensing is far more convenient than EMG sensing. Also, the solid-state nature of a PPG sensor is beneficial for use in the hostile environment of a patient's mouth, particularly in comparison with the often more complex structure of a pressure sensor.

Commonly, commercially available PPG sensors include an array of multiple LEDs, each emitting light in a different respective portion of the visible or invisible spectrum. In most cases, PPG sensors have three LEDs emitting light of respective wavelengths, namely red, green and IR. Thus, the optical sensor 46 of the sensor module 12 shown schematically in Figure 2 includes a red LED 52 and a green LED 54 in addition to the IR emitter 48 and the IR receiver 50.

Ambient light can interfere with the ability of a PPG sensor to measure muscle activity when placed externally on the body, hence the provision of LEDs emitting light at different wavelengths. However, as the invention is intended for use within the buccal cavity where ambient light levels are negligible, the IR emitter 48 alone is sufficient for measuring muscle activity. Another limitation on the use of a PPG sensor is that they are less effective at penetrating dark skin but this is generally not a problem within the buccal cavity.

In principle, it would be possible to use any other portion of the light spectrum instead of, or in addition to, IR but this is not essential and indeed could be disadvantageous for the purposes of the invention. As noted above, the PPG optical sensor 46 in the buccal cavity directed towards the masseter muscle 24 only requires one of the available light sources, namely the IR emitter 48, to measure activity of the masseter muscle 24. In this respect, IR light has been found to be advantageous for use within the buccal cavity as it is particularly effective at penetrating the mucus membrane and skin to sense the activity of muscles surrounding the buccal cavity. Also, limiting the number of LEDs used for operation of the optical sensor 46 is beneficial because it lowers the overall power requirement, reduces the complexity of the sensor module 12 and simplifies data processing.

Elegantly, whilst the red and green LEDs of a typical PPG sensor need not be used to sense activity of the masseter muscle 24, they can be used to indicate the status of the apparatus 10 and so are not redundant. For example, the green LED 54 could be used to indicate that the apparatus 10 is ready for use and the red LED 52 could be used to indicate that the battery 36 of the sensor module 12 is running low on charge.

PPG sensors can be sensitive to motion artefacts. In this respect, mounting the monitoring apparatus 10 on the upper jaw is advantageous as the upper jaw does not move relative to the masseter muscle 24 as a whole. This ensures resiliency to motion artefacts, repeatability of positioning and ease of calibration.

The housing 14 need not be in contact with the adjacent cheek 22 but any such contact between them, as may be inevitable, can be very gentle and light, exerting negligible pressure at the interface. Thus, the sensor unit 12 can monitor activity of the masseter muscle 24 without interfering with movement of the masseter muscle 24. The apparatus 10 also minimises discomfort, which could otherwise disturb the patient's sleep patterns and hence distort the measurements being taken to identify sleep bruxism events.

Information concerning activity of the masseter muscle 24 or other parameters that may be sensed in the oral vestibule 20 is collected by the sensor module 12 during a monitoring period. That information is transmitted wirelessly in a data signal by the transmitter 44 of the sensor module 12, for example via Bluetooth, to a remote receiver module or unit 56 shown in Figure 1. The sensor module 12 could stream the information to the receiver unit 56 in real time or could store and transmit the information to the receiver unit 56 periodically in one or more batches.

In some cases, the receiver unit 56 can conveniently comprise a mobile phone running an app with which a user can interact to request, interpret and receive information concerning activity of the masseter muscle 24 and hence bruxism events that may have arisen during a period of sleep. In this example, activity of the masseter muscle 24 over a period of time is shown in a graphical indication 58 on a screen 60 of the receiver unit 56 to show events such as a grind event 62 or a clench event 64, displayed here against the specific time of each event.

The sensor module 12 collects raw sensor data from within the oral vestibule 20 and can then process, or pre-process, the raw sensor data in its onboard processor 42, thereby minimising the amount of data to be transmitted to the receiver unit 56. The receiver unit 56 can also include a processor to process raw data, or to complete processing of partially processed data, received from the sensor module 12. The receiver unit 56 or the sensor module 12 can also delegate at least some processing duties to a processor in the cloud. One objective of such processing may be to filter out events involving activity of the masseter muscle 24 that are irrelevant to bruxism, such as yawning, the better to recognise activity of the masseter muscle 24 associated with bruxism events, such as a grind event 62 or a clench event 64.

The sensor module 12 can provide various measurements relating to the masseter muscle 24, some or all of which could be used to refine any measurement of muscle activity. For example, the sensor module 12 could provide a proximity measurement, up to and including the masseter muscle 24 touching the sensor module 12. Another measurement could be of blood volume changes giving an indication of masseter muscle activity. Moreover, changes in blood volume in the masseter muscle 24 can be used to interpret not just muscle activity but also heart rate (HR) and different sleep stages. Then, bruxing events can be identified from the HR, muscle activity and sleep stages. Muscle activity can also be used to determine of the patient's score on the apnea-hypopnea index (AHI). The sensor module 12 could also gather other measurements, for example relating to oxygen saturation (SpO2), heart rate variability (HRV) and barometric pressure.

As muscle physiology is incredibly complex, muscle shape may not change in some situations even while there is muscle activity, for example when holding a position. Thus, the sensor module 12 may be adapted or configured to measure one or both of muscle activity and change of muscle shape.

Activity of the masseter muscle 24 can also indicate or reflect the different stages of sleep. For example, there should be no voluntary muscle activity when a patient enters REM sleep, which means that any muscle activity during REM sleep is deemed involuntary and directly attributable to bruxing. Until now, the gold standard for measuring sleep bruxing has been masseter muscle activity measured using EMG correlated with sleep stages discerned from HR measurements. The present invention offers the possibility of being much more accurate than EMG in this respect.

The graph of Figure 3 plots the intensity of instances of bruxing against the intensity of sleep during a night-time period. Activity of the masseter muscle 24 detected and measured by the sensor module 12 can be used to characterise sleep stages and to characterise and quantify the intensity of movements of the jaw. Instances of clenching and grinding are shown here where they arise during alternating periods of light and deep sleep occurring between periods of wakefulness. Other detected jaw movements that are not indicative of bruxing, such as yawning, are also shown.

To position the sensor module 12 within the buccal cavity of a patient, the sensor module 12 can conveniently be attached to a thermoformed or otherwise moulded nightguard, being disposed on an outer surface and an outboard side of one limb of the nightguard. Alternatively, the nightguard can be constructed of layers with the sensor module 12 sandwiched between the layers, for example in an overmoulding process that forms an outer layer. In that case, the material of the outer layer must be sufficiently transparent or at least translucent to allow the optical sensor 46 to operate correctly.

The depth to which IR light emitted by the optical sensor 46 penetrates the tissue of the masseter muscle 24 can also be controlled by selecting the material surrounding the optical sensor 46 and the thickness of that material. For example, a wall of a structure covering the optical sensor 46 may be relatively thin where it covers the IR emitter 48 and IR receiver 50 and relatively thick where it otherwise surrounds and supports the body of the optical sensor 46. This combines good light transmission with structural strength required to position the optical sensor 46 accurately in the buccal cavity.

In another alternative, as shown in Figures 4 and 5, a custom nightguard 66 could be formed by additive manufacturing, in particular 3D printing. This enables the nightguard 66 to be tailored specifically for the wearer following an internal scan of the buccal cavity. In that case, the sensor module 12, or at least its housing 14, may conveniently be integrated into the same 3D printed component at the time of printing. Again, any material of the housing 14 covering the optical sensor 46 will be sufficiently transparent or translucent for the optical sensor 46 to function. This enables the sensor module 12 to be positioned correctly in the patient's buccal cavity while remaining totally enclosed and sealed or encapsulated within the housing 14.

3D printing can be used in various ways to manufacture a nightguard 66 like that shown in Figures 4 and 5. For example, the nightguard 66 could be printed with an interface feature on its outboard side, such as a flat surface or an interlocking formation, that facilitates bonding, welding or engaging an enclosure or housing 14 to or with the nightguard 66. Thus, advantageously, a standard sensor module 12, sealed within a standard housing 14, can be combined with a bespoke nightguard 66.

The sensor module 12 could be preinstalled in the housing 14 in a preliminary subassembly step before uniting the housing 14 with the 3D-printed nightguard 66. Alternatively, the sensor module 12 could be inserted into the housing 14 after the housing 14 is united with or formed with the nightguard 66, and then the housing 14 can be sealed around the sensor module 12. In this respect, Figure 5 shows that the housing 14 may be open-ended to receive the sensor module 12. The open end is then closed by a cap 68 that can be welded or bonded to the body of the housing 14 to encapsulate the sensor module 12. The cap 68 is shown here as a separate component but it could instead be formed integrally with the body of the housing 14 and, for example, hinged shut before the housing 14 is sealed around the sensor module 12.

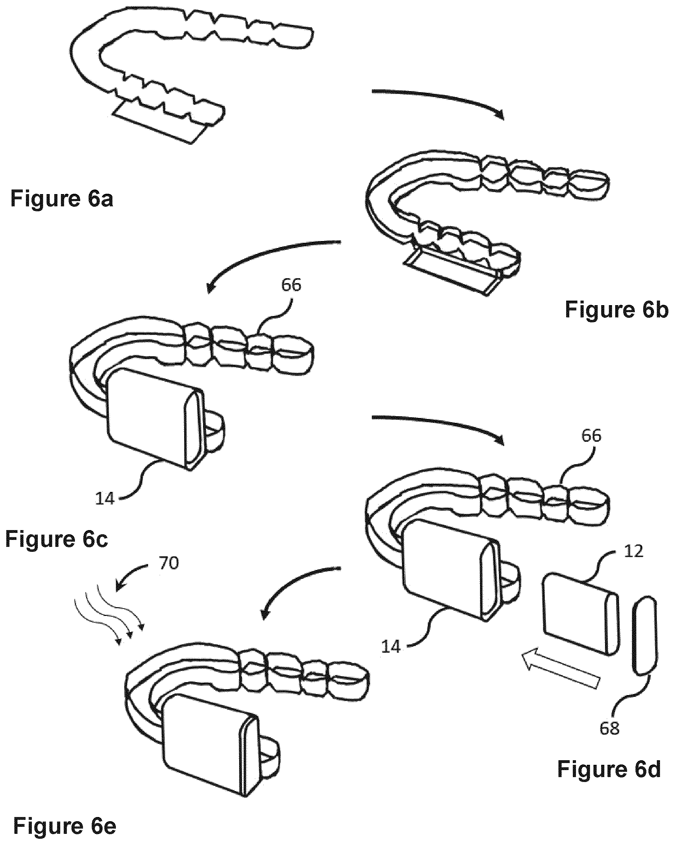

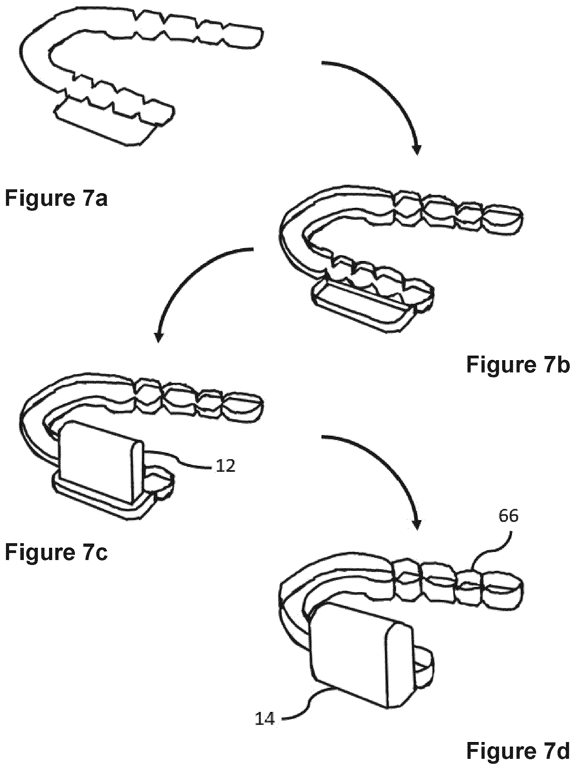

The sequences of Figures 6a to 6e and Figures 7a to 7d show, schematically, how the housing 14 can be formed simultaneously and integrally with the nightguard 66 as part of a single 3D printing process. In Figures 6a to 6e, an open-ended housing 14 is formed to receive the sensor module 12 after the printing process is complete, whereas in Figures 7a to 7d, the housing 14 is formed around the sensor module 12 during the printing process so that the sensor module 12 is already encapsulated when the printing process ends.

Figure 6a shows a base layer of the 3D-printed resin deposited on a printer bed and Figure 6b shows the 3D printing process continuing as layers of resin are built up, with the housing 14 being formed integrally with the nightguard 66. Figure 6c shows the printing process at an end, with the nightguard 66 in its final shape and the open-ended housing 14 completed.

Next, as shown in Figure 6d, the sensor module 12 is inserted into the housing 14 through the open end and the cap 68 is affixed to the housing 14 to close the open end. The cap 68 is then welded or bonded to the housing 14 to seal the sensor module 16 within. Bonding can be effected by applying the same resin used to form the nightguard 66 to the interface between the cap 68 and the housing 14 and then curing that resin by exposure to UV light 70 as shown in Figure 6e. Conveniently, this curing step can be performed during typical UV post-processing of the nightguard 66.

Correspondingly, Figures 7a and 7b show a base layer of resin deposited on a printer bed and the printing process continuing as layers of resin are built up. When the peripheral wall of the housing 14 formed integrally with the nightguard 66 is deep enough, the printing process is interrupted to allow the sensor module 12 to be placed within that peripheral wall, as shown in Figure 7c. The printing process is then resumed to continue forming the housing 14 around the sensor module 12, thus encapsulating the sensor module 12 within the completed housing 14 as shown in Figure 7d.

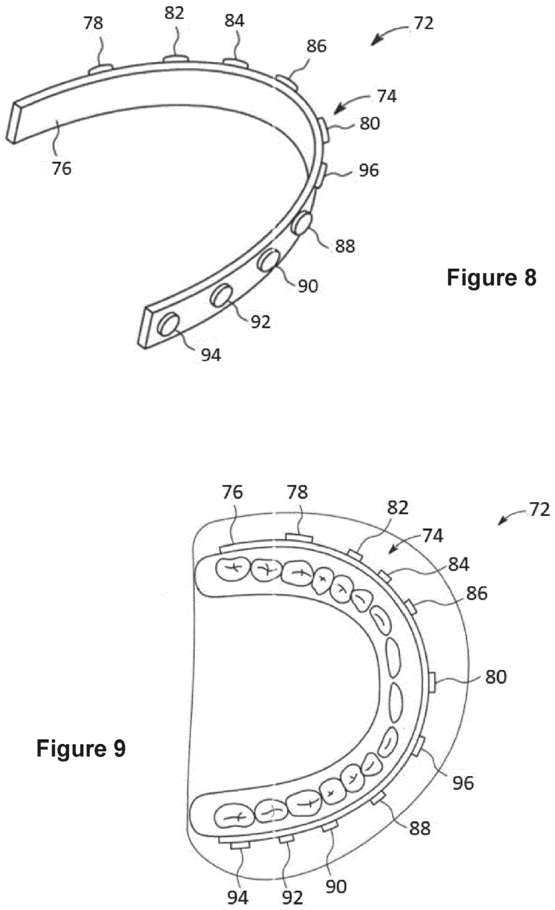

Turning finally to Figures 8 and 9, these drawings show a masseter muscle monitoring apparatus 72 in another embodiment of the invention.

The apparatus 72 comprises a sensor module 74 mounted on a support 76 for mounting the apparatus 72 within the oral vestibule 20. In this case, the sensor module 74 has an array of sensors including a first PPG optical sensor 78 positioned on the support 76 so that, in use, it faces outwardly toward the masseter muscle when fixed relative to a patient's upper jaw. Various other sensors are spaced apart around or along the support 76, these including a second PPG optical sensor 80 that locates elsewhere in the oral vestibule 20 or buccal cavity to obtain blood pressure measurements in combination with the first PPG optical sensor 78. The second PPG optical sensor 80 could alternatively be mounted elsewhere on the body, for example on a smart watch worn on the patient's wrist.

Other sensors mounted on the support 76 include a temperature sensor 82, a pressure sensor 84, an accelerometer 86, and a pH sensor 88. Also mounted on the support 76 are a microphone 90, a battery 92, charging circuitry 94 and a Bluetooth module 96. The support 76 suitably comprises a hybrid circuit board.

It will be appreciated that apparatus of the invention can be used not only with humans but also with other mammals such as horses and cows, where it can be used to measure muscle activity showing chewing and indicative of digestion. This can be complemented with other measurements, such as HR, SpO2, HRV, sleep stages and temperature, to help to assess the health of the animal or herd. It is envisaged that the data could be collected from a herd using the networking features of a protocol like Bluetooth. In the case of cows, the device would more likely be bonded to or otherwise attached directly to an outer side of an upper back tooth or lower back tooth.

The invention is not limited to the embodiments hereinbefore described, which may be varied in both construction and detail. Among possible variations within the inventive concept, multiple sensor modules 12 could be provided within the buccal cavity if desired, for example one sensor module 12 in the oral vestibule 20 on each side of the buccal cavity to sense activity of each masseter muscle 24. This could enrich the data being collected, allowing comparison of activity between the opposed masseter muscles 24 during a bruxism event. In this respect, it is known that bruxism events can be asymmetric across the masseter muscles 24 of a patient.

If provided in a single apparatus, multiple sensor modules 12 could be connected to each other within the apparatus, for example by wires extending around the arcuate body of a nightguard or other oral accessory. This would enable the sensor modules 12 to share facilities onboard the apparatus, such as a power supply or a data transmitter. Such facilities could be hosted by either or both of the sensor modules 12 or could be mounted separately from the sensor modules 12, for example at a position between the sensor modules 12 and therefore adjacent to the patient's front teeth or incisors.

It is known to use a PPG sensor to measure SpO2, HR and HRV. However, with current PPG technology, such measurements cannot be taken effectively with a PPG sensor directed towards the masseter muscle. The optimal place for the PPG sensor in the buccal cavity in this case may be at the front of the buccal cavity above the gum line. Another embodiment of the invention could therefore involve the use of a second PPG sensor positioned at the front of the buccal cavity above the gum line, with the intent of measuring SpO2, HR and/or HRV.

It is also envisioned that an optical sensor 46 could operate in combination with other sensors, for example a temperature sensor, an accelerometer or a gyroscope. Data from an accelerometer and/or a gyroscope included in the sensor module 12 can be combined with data from the optical sensor 46 to help to differentiate the different clenching and grinding muscle activities that occur. Movements and changes in the orientation of the patient's head and body during sleep can also be determined, which may be insightful in the measurement and analysis of bruxism events.

Mounting a monitoring apparatus 10 of the invention on the lower jaw or mandible could also be desirable and advantageous in some circumstances. For example, data from an optical sensor 46 combined with data from an accelerometer could better differentiate between clenching and grinding and other mouth actions such as yawning and chewing. Indeed, a monitoring apparatus 10 of the invention can be mounted on or engaged with either or both the upper jaw and the lower jaw. In the letter case, the flows of data produced by the respective devices can be processed differently as required, for example to corroborate indications of masseter muscle activity with each other. Indications of masseter muscle activity can also be correlated with other inputs such as patient movement, orientation, temperature or other physiological indications such as SpO2 or HR.

In addition to an optical sensor 46 in the buccal cavity directed toward the masseter muscle 24, another sensor - whether optical or otherwise - can be located elsewhere on or in the body. For example, it is envisaged that a second sensor could be adapted for mounting in a patient's ear instead of or in addition to a sensor module 12 within the buccal cavity. A second sensor could also take the form of a wearable smart device with PPG or other sensor capability, for example a smart watch, a smart bracelet or a smart ring device such as the 'O̅ura' ring (registered trade mark) as sold by Oura Health Oy of Finland. Additional sensors like these would, for example, allow continuous monitoring of blood pressure and could transmit their signals to the same remote receiver unit 56 as the sensor module 12.

Many other modifications or alternatives are possible within the inventive concept. For example, the apparatus of the invention could include a feedback component to provide haptic feedback and/or sound feedback. If desired, haptic and/or sound feedback could be provided to a user during a bruxing event so as to interrupt the event. As the device is attached to a tooth, vibration is particularly effective at wakening the patient.

The position of the sensor module 12 within the buccal cavity and the direction in which it acts is important for the masseter muscle 46 being monitored by the apparatus of the invention. In this regard, the sensor module 12 could configure or adjust the direction in which it acts either by having multiple light sources, such as multiple IR LEDs, or by focusing or directing the light emitted by the optical sensor 46 in different directions. For example, the direction in which light is emitted from the optical sensor 46 could be controlled by lensing to aim at an area considered optimal to measure masseter muscle activity.

Whilst the focus of the invention is to monitor the masseter muscle 46 and to use the information thereby collected to monitor bruxing, it is also envisaged that the collected information could be used to monitor another condition or state of a patient. Also, the apparatus could be adapted to control the delivery of medication to the patient, for example by injection through micro needles into the vascular structure surrounding the buccal cavity.

## Claims

1. A method of monitoring masseter muscle activity from a location within a wearer's buccal cavity, based on monitoring data obtained from an optical sensor the method comprising:
placing said optical sensor (46) into the buccal cavity, facing the masseter muscle (24), at a position fixed relative to at least one tooth adjacent to the masseter muscle (24);
emitting light from the optical sensor (46) into the masseter muscle (24);
detecting reflection of the light from the masseter muscle (24) into the optical sensor (46);
obtaining monitoring data from the detected light, indicative of activity of the masseter muscle (24); and
wirelessly transmitting the monitoring data to a remote receiver unit (56).

2. The method of Claim 1, comprising emitting IR light from the optical sensor (46) into the masseter muscle (24).

3. The method of any preceding claim, comprising determining the wearer's sleep or activity states by analysing the monitoring data.

4. The method of Claim 3, comprising determining bruxism activity by analysing the monitoring data, and correlating the bruxism activity with the determined sleep or activity states.

5. The method of any of Claims 2 to 4, comprising:
placing an additional sensor onto, or into an orifice of, the wearer's body, that additional sensor being one or more of an optical sensor (46), an accelerometer (86), a gyroscope, a temperature sensor (82), a pressure sensor (84), a humidity sensor and a pH sensor (88); and
wirelessly transmitting additional data from the or each additional sensor to the remote receiver unit (56).

6. The method of Claim 5, comprising placing the or each additional sensor into the buccal cavity together with the optical sensor as a single unit.

7. The method of any preceding claim, comprising:
Placing an additional optical sensor (46) into the buccal cavity facing another masseter muscle (24);
obtaining additional monitoring data indicative of activity of that other masseter muscle (24);
wirelessly transmitting the additional monitoring data to a remote receiver unit (56); and
comparing the monitoring data with the additional monitoring data.

## Patentansprüche

1. Verfahren zur Überwachung von Aktivität des Musculus Masseter von einer Position innerhalb einer Mundhöhle eines Trägers, basierend auf Überwachungsdaten, die von einem optischen Sensors erhalten wurden,
wobei das Verfahren Folgendes umfasst:
Positionieren des optischen Sensors (46) in der Mundhöhle zum Musculus Masseter (24) zeigend, an einer festen Position in Bezug auf mindestens einen Zahn angrenzend zu dem Musculus Masseter (24);
Emittieren von Licht von dem optischen Sensor (46) in den Musculus Masseter (24);
Detektieren von Reflexion des Lichts von dem Musculus Masseter (24) in den optischen Sensor (46);
Erhalten von Überwachungsdaten aus dem detektierten Licht, die Aktivität des Musculus Masseter (24) angeben; und
drahtloses Übertragen der Überwachungsdaten an eine entfernte Empfängereinheit (56).

2. Verfahren nach Anspruch 1, umfassend Emittieren von IR-Licht von dem optischen Sensor (46) in den Musculus Masseter (24).

3. Verfahren nach einem vorstehenden Anspruch, umfassend Bestimmen des Schlaf- oder Aktivitätszustands des Trägers durch Analysieren der Überwachungsdaten.

4. Verfahren nach Anspruch 3, umfassend Bestimmen von Bruxismus-Aktivität durch Analysieren der Überwachungsdaten und Korrelieren der Bruxismus-Aktivität mit dem bestimmten Schlaf- oder Aktivitätszustand.

5. Verfahren nach einem der Ansprüche 2 bis 4, umfassend:
Positionieren eines zusätzlichen Sensors auf oder in einer Öffnung des Körpers des Trägers, wobei der zusätzliche Sensor eines oder mehreres ist von einem optischen Sensor (46), einem Beschleunigungsmesser (86), einem Gyroskop, einem Temperatursensor (82), einem Drucksensor (84), einem Feuchtigkeitssensor und einem pH-Sensor (88); und
drahtloses Übertragen von zusätzlichen Daten von dem oder jedem zusätzlichen Sensor an die entfernt Empfängereinheit (56).

6. Verfahren nach Anspruch 5, umfassend Positionierten des oder jedes zusätzlichen Sensor in der Mundhöhle zusammen mit dem optischen Sensor als eine einzige Einheit.

7. Verfahren nach einem vorstehenden Anspruch, umfassend:
Positionieren eines zusätzlichen optischen Sensors (46) in der Mundhöhle, zu einem anderen Musculus Masseter (24) zeigend;
Erhalten von zusätzlicher Überwachungsdaten, die Aktivität des anderen Musculus Masseter (24) angeben;
drahtloses Übertragen der zusätzlichen Überwachungsdaten an eine entfernte Empfängereinheit (56); und
Vergleichen der Überwachungsdaten mit den zusätzlichen Überwachungsdaten.

## Revendications

1. Procédé de surveillance de l'activité du muscle masséter à partir d'un emplacement dans la cavité buccale d'un utilisateur, sur la base de données de surveillance obtenues à partir d'un capteur optique
le procédé comprenant :
la mise en place dudit capteur optique (46) dans la cavité buccale, en regard du muscle masséter (24), à une position fixée par rapport à au moins une dent adjacente au muscle masséter (24) ;
l'émission de lumière à partir du capteur optique (46) dans le muscle masséter (24) ;
la détection de la réflexion de la lumière du muscle masséter (24) dans le capteur optique (46) ;
l'obtention de données de surveillance à partir de la lumière détectée, indiquant l'activité du muscle masséter (24) ; et
la transmission sans fil des données de surveillance à une unité réceptrice distante (56).

2. Procédé selon la revendication 1, comprenant l'émission de lumière IR à partir du capteur optique (46) dans le muscle masséter (24).

3. Procédé selon une quelconque revendication précédente, comprenant la détermination des états de sommeil ou d'activité de l'utilisateur en analysant les données de surveillance.

4. Procédé selon la revendication 3, comprenant la détermination de l'activité de bruxisme en analysant les données de surveillance, et la corrélation de l'activité de bruxisme avec les états de sommeil ou d'activité déterminés.

5. Procédé selon l'une quelconque des revendications 2 à 4, comprenant :
la mise en place d'un capteur supplémentaire sur, ou dans un orifice du corps de l'utilisateur, ce capteur supplémentaire étant un ou plusieurs parmi un capteur optique (46), un accéléromètre (86), un gyroscope, un capteur de température (82), un capteur de pression (84), un capteur d'humidité et un capteur de pH (88) ; et
la transmission sans fil de données supplémentaires du ou de chaque capteur supplémentaire à l'unité réceptrice distante (56).

6. Procédé selon la revendication 5, comprenant la mise en place du ou de chaque capteur supplémentaire dans la cavité buccale avec le capteur optique comme une seule unité.

7. Procédé selon une quelconque revendication précédente, comprenant :
la mise en place d'un capteur optique (46) supplémentaire dans la cavité buccale en regard d'un autre muscle masséter (24) ;
l'obtention de données de surveillance supplémentaires indiquant l'activité de cet autre muscle masséter (24) ;
la transmission sans fil des données de surveillance supplémentaires à une unité réceptrice distante (56) ; et
la comparaison des données de surveillance avec les données de surveillance supplémentaires.
